# EUROPEAN PATENT APPLICATION

(11) **EP 2 773 102 A2**
(43) Date of publication of application: **03.09.2014**
(21) Application number: 14153092.3
(22) Date of filing: 29.01.2014
(51) Int. Cl.: H04N 5/32, G01T 1/24

(54) **Radiation imaging apparatus, radiation inspection apparatus, method for correcting signal, and program**

(30) Priority: 28.02.2013 JP 2013040035
(71) Applicant: CANON KABUSHIKI KAISHA, Ohta-ku Tokyo 146-8501 (JP)
(72) Inventor: Sato, Eriko, Ohta-ku, Tokyo (JP); Kameshima, Toshio, Ohta-ku, Tokyo (JP); Yagi, Tomoyuki, Ohta-ku, Tokyo (JP); Takenaka, Katsuro, Ohta-ku, Tokyo (JP); Okada, Hideyuki, Ohta-ku, Tokyo (JP); Sato, Sho, Ohta-ku, Tokyo (JP); Iwashita, Atsushi, Ohta-ku, Tokyo (JP); Ryu, Takuya, Ohta-ku, Tokyo (JP)
(74) Representative: Garner, Jonathan Charles Stapleton

(57) **Abstract**

A radiation imaging apparatus (100), comprising a pixel array (101) on which a plurality of pixels are arrayed, a readout unit (103) configured to read out a signal from the pixel array, a first unit (116) configured to specify a portion of the signal read out by the readout unit, at which a saturated signal whose value has reached a saturation level of an output by the readout unit and a non-saturated signal whose value has not reached the saturation level are mixed, and a second unit (117) configured to correct, based on the non-saturated signal, the saturated signal at the portion specified by the first unit.

## Description

### BACKGROUND OF THE INVENTION

### Field of the Invention

The present invention relates to a radiation imaging apparatus, a radiation inspection apparatus, a method for correcting a signal, and a program.

### Description of the Related Art

A radiation imaging apparatus used in a radiation inspection apparatus can include a pixel array configured to array a plurality of pixels on an insulating substrate, and a readout unit configured to read out a signal from the pixel array.

For example, at a portion of the pixel array that is irradiated with radiation at a high intensity, a signal value from the pixel may exceed the upper limit of a value outputtable from the pixel or exceed a voltage range processible by the readout unit. That is, the signal of the pixel reaches the saturation level.

Japanese Patent Laid-Open No. 2003-576 discloses a radiation imaging apparatus in which it is determined whether the signal of each pixel has reached the saturation level, and as for each signal which has reached the saturation level, its value is fixed to a predetermined value. In Japanese Patent Laid-Open No. 2003-576, each signal which has reached the saturation level does not contain variations (correction noise) generated by correcting a signal from a pixel.

A signal near the saturation level is obtained in, for example, a region of the pixel array where radiation not having passed through an object X is detected. However, when noise components of different amounts are mixed in respective pixels of the region, saturated and non-saturated signals are mixed. More specifically, even if radiation enters two adjacent pixels at the same dose, one pixel may output a saturated signal and the other pixel may output a non-saturated signal.

However, in Japanese Patent Laid-Open No. 2003-576, the value of each signal which has reached the saturation level is fixed to a predetermined value, so the continuity between adjacent signals is lost. This may appear as a striped artifact in a radiation image formed based on the signals and may hinder inspection or diagnosis.

### SUMMARY OF THE INVENTION

The present invention provides a technique advantageous for improving the quality of an image obtained by a radiation imaging apparatus.

The present invention in its first aspect provides a radiation imaging apparatus, as specified in claims 1 to 7.

The present invention in its second aspect provides a radiation inspection apparatus, as specified in claim 8.

The present invention in its third aspect provides a method for correcting a signal, as specified in claim 9.

The present invention in its fourth aspect provides a program, as specified in claim 10.

The present invention in its fifth aspect provides a storage medium, as specified in claim 11.

Further features of the present invention will become apparent from the following description of exemplary embodiments with reference to the attached drawings.

### BRIEF DESCRIPTION OF THE DRAWINGS

Fig. 1 is a circuit diagram for explaining an example of the arrangement of a radiation imaging apparatus;
Fig. 2 is a block diagram for explaining an example of the arrangement of a radiation inspection apparatus;
Fig. 3 is a chart for explaining an example of the operation sequence of the radiation imaging apparatus;
Figs. 4A and 4B show examples of the plots of signals obtained by the radiation imaging apparatus;
Fig. 5 is a flowchart for explaining an example of a method for correcting a signal obtained by the radiation imaging apparatus;
Figs. 6A and 6B show examples of the plots of signals obtained by the radiation imaging apparatus;
Fig. 7 shows an example of the plots of signals obtained by the radiation imaging apparatus;
Fig. 8 is a chart for explaining an example of the operation sequence of the radiation imaging apparatus; and
Figs. 9A and 9B show examples of the plots of signals obtained by the radiation imaging apparatus.

### DESCRIPTION OF THE EMBODIMENTS

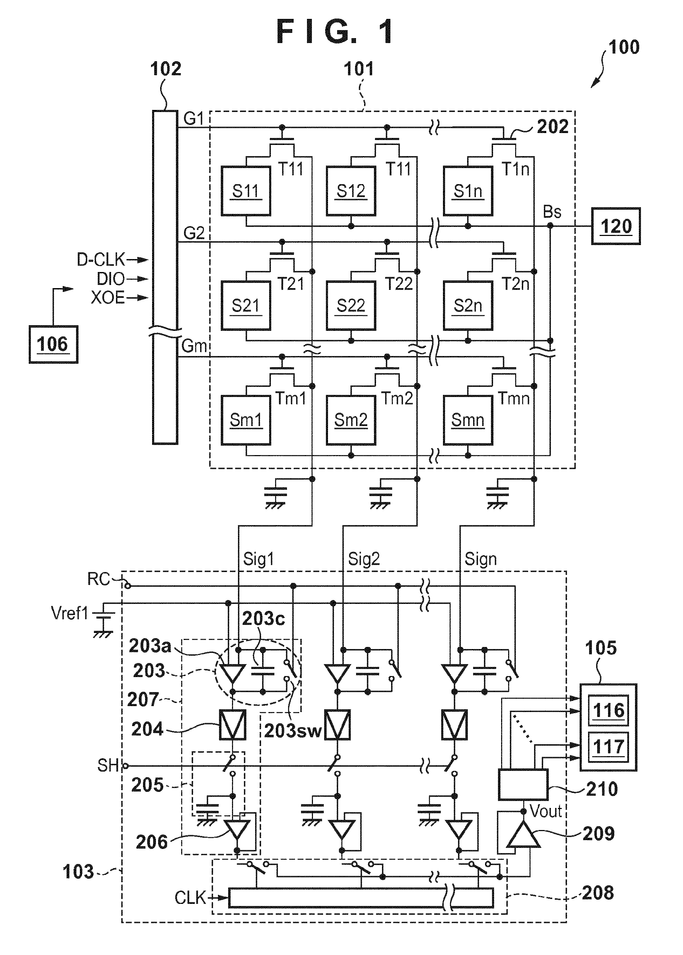

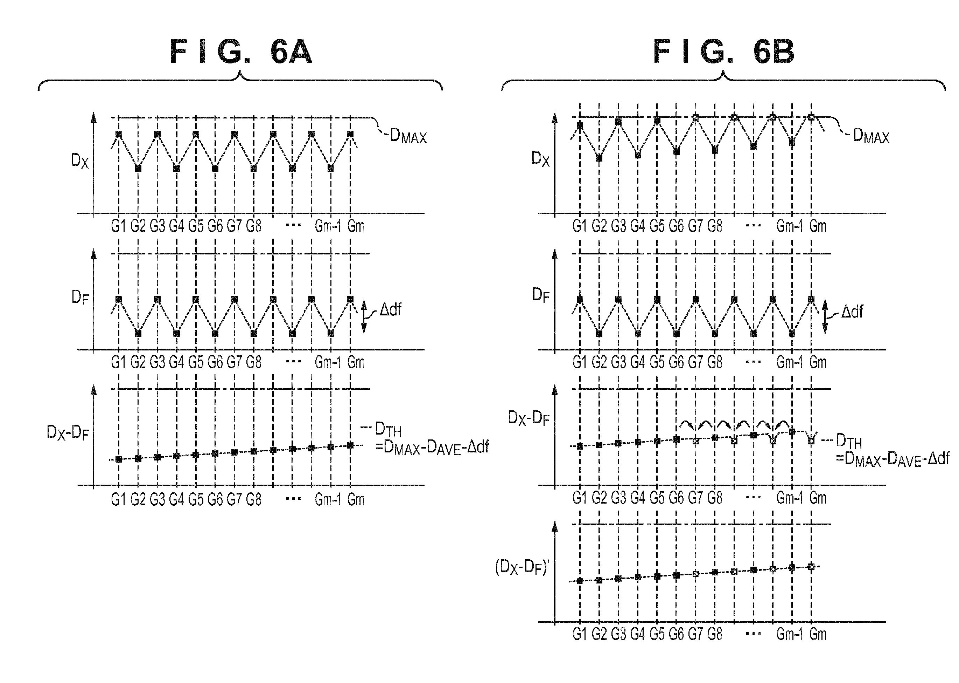

A radiation imaging apparatus 100 according to the present invention will be explained with reference to Figs. 1 to 6A and 6B. As exemplified in Fig. 1, the radiation imaging apparatus 100 can include a sensor unit 101 (pixel array) configured to array a plurality of pixels P, a driving unit 102 configured to drive the sensor unit 101, a readout unit 103 configured to read out a signal from the sensor unit 101, a processing unit 105, and a control unit 106.

The sensor unit 101 is constructed by forming m (row) x n (column) pixels P, that is, P₁₁ to Pₘₙ. Although 3 (row) x 3 (column) pixels are shown, pixels of several thousand rows x pixels of several thousand columns are formed in practice. For example, about 2,800 (row) x 2,800 (column) pixels are formed for a 17-inch size. The respective pixels P can include conversion elements S, that is, S₁₁ to Sₘₙ configured to output a signal corresponding to incident radiation, and switching elements T, that is, T₁₁ to Tₘₙ connected to the corresponding conversion elements S. The switching elements T are interposed between the corresponding conversion elements S and signal lines Sig, that is, Sig₁ to Sigₙ arranged in correspondence with the respective columns. When the switching element T is turned on, a signal from the conversion element S is output to the readout unit 103 via the signal line Sig. It is only necessary to configure the sensor unit 101 to detect incident radiation by each pixel P, and the sensor unit 101 can adopt a known arrangement. For example, the sensor unit 101 can be formed on an insulating substrate such as a glass substrate, a sensor such as a PIN sensor or MIS sensor can be used as the conversion element S, and a scintillator layer (layer for converting radiation into light) can be arranged on the conversion element S. The conversion element S may be configured to directly convert incident radiation into an electrical signal. As the switching element T, a TFT (Thin Film Transistor) is usable.

The driving unit 102 drives the sensor unit 101 in accordance with control signals (for example, D-CLK, DIO, and XOE) from the control unit 106. For example, the control unit 106 controls the driving unit 102 to output a signal from the conversion element S to the readout unit 103 via the signal line Sig by turning on the switching element T, or initialize (reset) the conversion element S, details of which will be described later. Note that the control signal D-CLK is the shift clock of a shift register constructing the driving unit 102. The control signal DIO is a pulse transferred from the shift register. The control signal XOE is the output enable signal of the shift register.

The radiation imaging apparatus 100 can further include a bias line Bs configured to supply a predetermined bias to (the sensor S of) each pixel P, and a detection unit 120 configured to monitor the current amount of the bias line Bs and detect irradiation with radiation based on the change amount of the current amount. The detection unit 120 can be constructed using, for example, a current-to-voltage conversion means for converting a current on the bias line Bs into a voltage, and a determination means for determining whether the converted voltage has reached a reference value. With this arrangement, in response to irradiation with radiation, the radiation imaging apparatus 100 can start a readout operation of reading out a signal from each conversion element S.

The readout unit 103 suffices to read out signals from the sensor unit 101 sequentially for the respective columns, and can employ a known arrangement. The readout unit 103 can include, for example, amplification units 207, a multiplexer 208, a buffer amplifier 209, and an A/D converter 210. The amplification units 207 are arranged in correspondence with the respective columns, and amplify signals from the sensor unit 101. Each amplification unit 207 can include at least one amplification means (for example, an integrating amplifier 203, variable amplifier 204, and buffer amplifier 206), and a sample and hold circuit 205. In the arrangement, a signal propagating through the signal line Sig is amplified by the integrating amplifier 203 and variable amplifier 204.

Note that the integrating amplifier 203 can be constructed using, for example, an operational amplifier 203a, integral capacitor 203c, and reset switch 203sw. One input terminal of the operational amplifier 203a can be connected to the signal line Sig, and its other input terminal can be connected to (the node of) a reference potential Vref1. It suffices to determine the value of the integral capacitor 203c in accordance with a target amplification factor or specification. The switch 203sw resets the potential of the input/output node in response to a control signal RC.

A signal amplified by the integrating amplifier 203 and variable amplifier 204 can be sampled in accordance with a control signal SH in the sample and hold circuit 205 constructed by, for example, a switch and capacitor. Sampled signals are sequentially output to the buffer amplifier 209 via the buffer amplifier 206 from the multiplexer 208 which operates in accordance with a control signal CLK. A signal from the multiplexer 208 undergoes impedance conversion by the buffer amplifier 209 and then is output to the A/D converter 210, obtaining a digital signal.

The signal read out in this manner by the readout unit 103 undergoes, for example, predetermined processing in the processing unit 105, and a radiation image can be formed. The processing unit 105 can include a determination unit 116 (first unit) and correction unit 117 (second unit). The determination unit 116 determines and specifies a portion which requires correction in a signal obtained from the sensor unit 101. The correction unit 117 performs correction processing for the portion requiring correction. Details of the determination unit 116 and correction unit 117 will be described in embodiments to be described later.

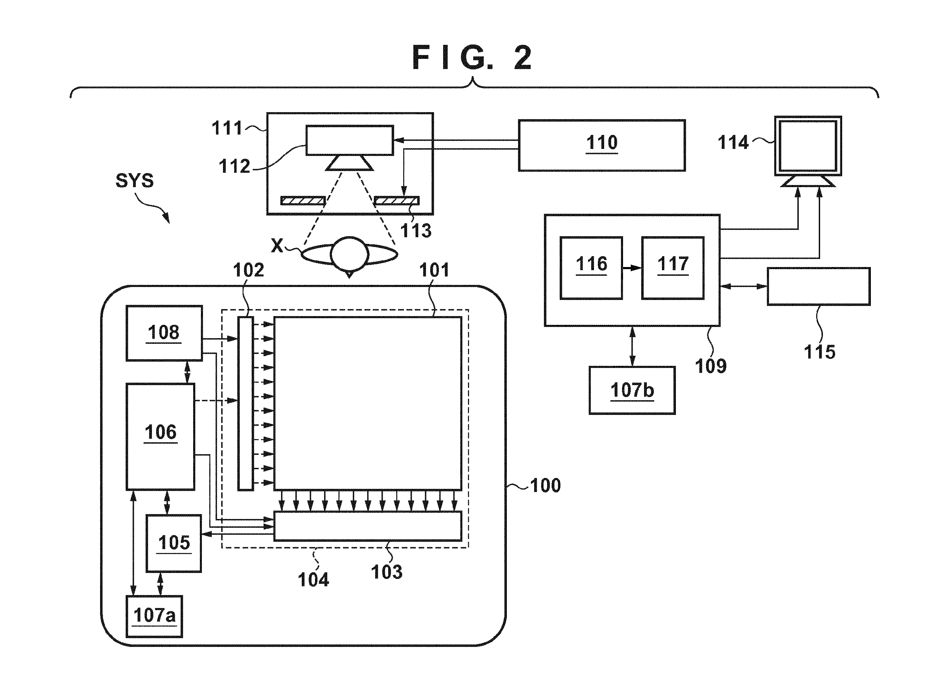

As exemplified in Fig. 2, the radiation imaging apparatus 100 is applicable to, for example, a radiation inspection apparatus SYS (imaging system) which inspects an object X by radiation (including an X-ray, α-ray, β-ray, and γ-ray). In addition to the radiation imaging apparatus 100, the radiation inspection apparatus SYS can include, for example, a radiation generation apparatus 111 for generating radiation, an external processing unit 109, and a display unit 114 such as a display. The radiation generation apparatus 111 can include, for example, a radiation source 112 and irradiation region aperture mechanism 113. In response to an input from an emission switch 110, the radiation source 112 emits radiation. The emitted radiation passes through the object X and is detected by the radiation imaging apparatus 100. The detected radiation contains information about the inside of the body of the object X. The radiation imaging apparatus 100 acquires a signal based on this radiation. The acquired signal undergoes predetermined signal processing in the processing unit 105, and is transferred to the external processing unit 109 via, for example, wireless communication units 107a and 107b. After that, the display unit 114 can display a radiation image based on the acquired signal in accordance with an input from an operation unit 115.

### (First Embodiment)

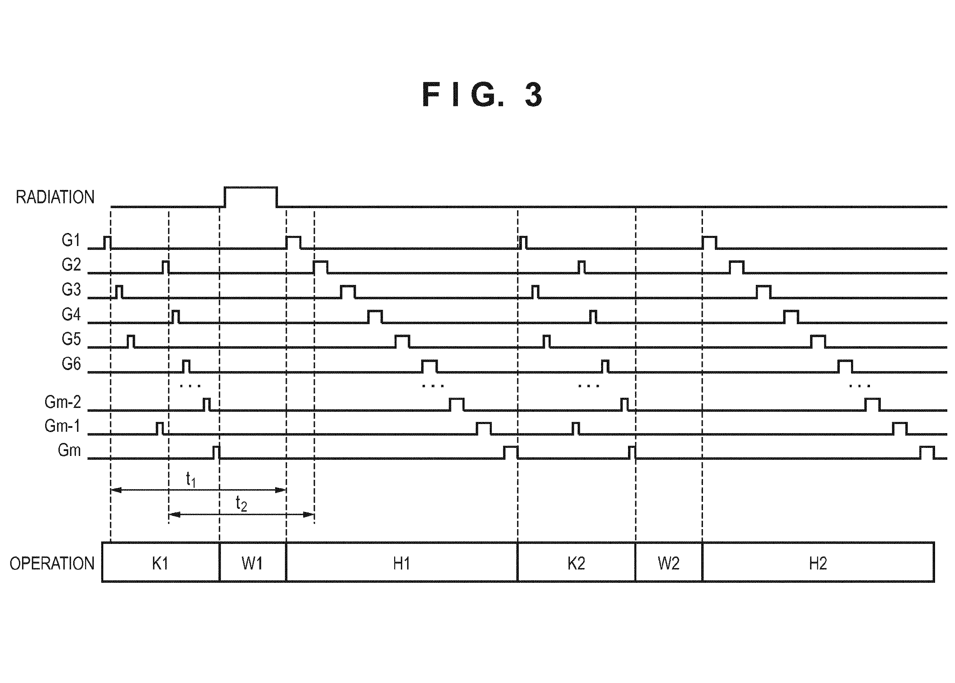

First, the operation sequence of a radiation imaging apparatus 100 will be described. As exemplified in Fig. 3, a control unit 106 controls a driving unit 102 to mainly perform a reset operation, standby operation, and readout operation. In the reset operation, for example, switching elements T can be turned on sequentially for the respective rows in accordance with a control signal from the driving unit 102, and respective pixels can be cyclically reset for every row. In the reset operation, a signal line Sig is fixed to a reference potential Vref1, and when the switching element T is turned on, a conversion element S is initialized.

In the first embodiment, the reset operation can be performed by alternately repeating resetting of pixels arranged on odd-numbered rows G1, G3, G5,..., Gm-1 in a sensor unit 101, and resetting of pixels arranged on even-numbered rows G2, G4, G6,..., Gm. According to this reset method, for example, when large noise is mixed in one signal (or one row) among signals obtained from the sensor unit 101, the noise-containing signal can be corrected using signals obtained from pixels adjacent to a pixel corresponding to this signal.

In the standby operation, for example, the switching elements T in respective pixels P are kept OFF for a predetermined period to accumulate charges in the conversion elements S. In the readout operation, the switching elements T are turned on sequentially for the respective rows in accordance with a control signal from the driving unit 102, and signals (signals of the respective pixels P) corresponding to the amounts of charges accumulated in the conversion elements S are output to a readout unit 103 sequentially for the respective rows.

Fig. 3 shows the operation sequence of the radiation imaging apparatus 100. Fig. 3 shows the irradiation dose of radiation, control signals input to the switching elements T on the respective rows G1, G2,..., Gm, and the operation mode of the radiation imaging apparatus 100. For example, after the radiation imaging apparatus 100 is activated, a reset operation K1 (first reset operation) can be performed. In the reset operation according to the embodiment, the pulse width of the control signal input to turn on the switching elements T on each row suffices to be set to about 16 µs.

In response to irradiation with radiation, the reset operation K1 is interrupted, and a standby operation W1 is performed to stand by for a predetermined period. In the period of the standby operation W1, charges generated in accordance with incident radiation are accumulated in each pixel. As described above, a detection unit 120 can detect irradiation with radiation based on the change amount of the current amount on the bias line Bs.

After the standby operation W1, a readout operation H1 (first readout operation) can be performed. In the readout operation according to the embodiment, the pulse width of the control signal input to turn on the switching elements T on each row suffices to be set to about 50 µs. As a result, a signal from each pixel P of the sensor unit 101 is output to the readout unit 103.

In response to the end of the readout operation H1, a reset operation K2 (second reset operation) can start. At this time, the reset operation K2 is performed for the time of at least one cycle of the reset operation K2. That is, each pixel P is reset at least once. After the lapse of the time of at least one cycle, the reset operation K2 can be interrupted on a row on which the reset operation K1 was interrupted. After that, a standby operation W2 is performed to stand by for the same period as that of the standby operation W1. Then, a readout operation H2 (second readout operation) can be performed.

The processing unit 105 can perform processing of, for example, calculating a difference between a signal obtained by the readout operation H1 and a signal obtained by the readout operation H2. The reset operation K2 is interrupted on a row on which the reset operation K1 was interrupted, and the period of the reset operation K1 to the readout operation H1 and the period of the reset operation K2 to the readout operation H2 become equal to each other on each row. Hence, for each row, a noise component (including an offset component) corresponding to the row from which a signal has been read out by the readout operation H2 can be subtracted from a signal component read out by the readout operation H1. In this manner, a signal is obtained as a radiation image.

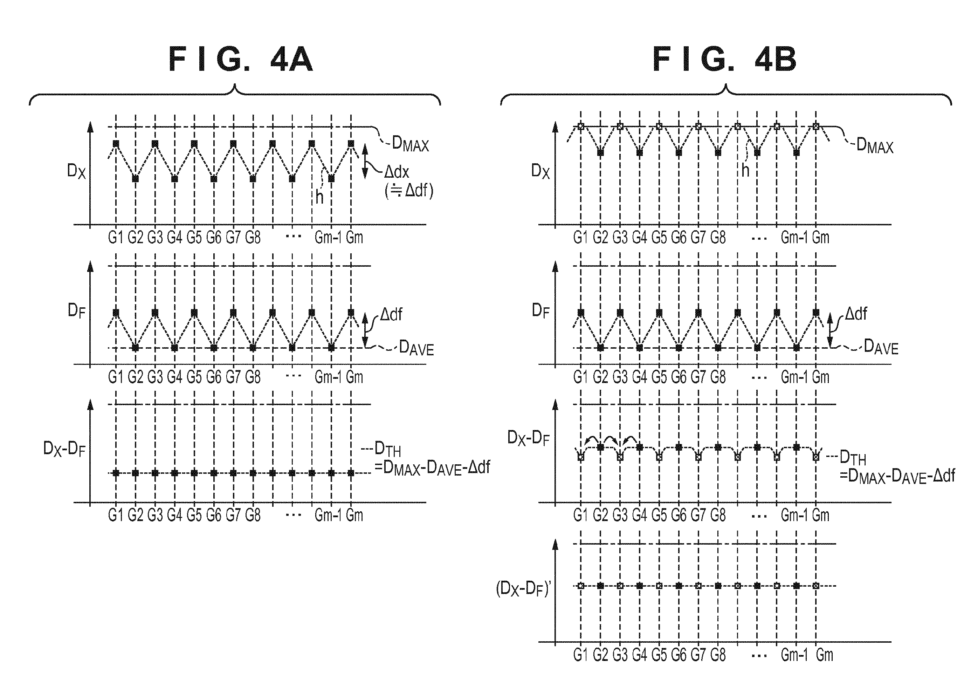

Next, signals obtained from the sensor unit 101 by a sequence of performing the above-described reset operation (that is, the reset operation of alternately repeating resetting of pixels arranged on odd-numbered rows and resetting of pixels arranged on even-numbered rows) will be described with reference to Figs. 4A and 4B. For descriptive convenience, irradiation with radiation at a uniform irradiation dose will be examined.

Fig. 4A shows the plots of a signal D_{X} obtained by the readout operation H1, a signal D_{F} obtained by the readout operation H2, and a signal D_{X}-D_{F} calculated by the processing unit 105 for the respective rows upon uniform irradiation with radiation at a low intensity. For example, when the reset operation K1 (and K2) is interrupted on an even-numbered row (in Fig. 3, it ends on the mth row Gm), the time of the reset operation K1 to the readout operation H1 (or the reset operation K2 to the readout operation H2) is longer on an odd-numbered row than that on an even-numbered row. That is, t1 > t2, as shown in Fig. 3. For this reason, the plots of the signals D_{X} and D_{F} represent larger values on odd-numbered rows than on even-numbered rows. Note that a dotted line h in Fig. 4A indicates that the rise and fall of the plot are repeated owing to the difference in the time of the reset operation K1 to the readout operation H1 (or the reset operation K2 to the readout operation H2) between odd-numbered rows and even-numbered rows.

Letting Δdx (absolute value) be the average value of the difference in signal D_{X} between odd-numbered rows and even-numbered rows, and Δdf (absolute value) be the average value of the difference in signal D_{F} between odd-numbered rows and even-numbered rows, Δdx and Δdf become almost equal. Thus, the signal D_{X}-D_{F} draws an almost uniform plot. As is apparent from Fig. 3, the time of one cycle of the reset operation is shorter than the time taken for one readout operation in practice. Therefore, the time of the reset operation K1 to the readout operation H1 (or the reset operation K2 to the readout operation H2) becomes longer for a larger row G number. Although the signal D_{X}-D_{F} is represented to draw an almost uniform plot, it can have shading practically.

In some cases, a signal read out from the sensor unit 101 to the readout unit 103 contains not only a non-saturated signal whose value has not reached the saturation level of an output from the readout unit 103, but also a saturated signal whose value has reached the saturation level. In this case, the state in which the value of a signal has reached the saturation level can include a state in which an output value from the readout unit 103 has reached the maximum value of an outputtable value from the readout unit 103 in the arrangement of the radiation imaging apparatus 100. As an example in which a signal reaches the saturation level, a large noise component is read out and exceeds the input voltage range or operation range of the readout unit 103 when no reset processing of the sensor unit 101 has been performed for a predetermined period. As another example, the sensor unit 101 is irradiated with radiation at a high intensity to generate a large amount of charges in the conversion element, and the signal exceeds a range which can be handled by the pixel P serving as a sensor. In both of the above-described cases, output values from the readout unit 103 can become almost the same.

Similar to Fig. 4A, Fig. 4B shows the plots of the signal D_{X}, the signal D_{F}, the signal D_{X}-D_{F}, and a signal (D_{X}-D_{F})' after performing correction processing by the correction unit 117 upon uniform irradiation with radiation at a high intensity. As for the signal D_{X}, the plot reaches a saturation level D_{MAX} on odd-numbered rows. As for the signal D_{F}, the plot is the same as that in the case in which the intensity is low (Fig. 4A). Since a component corresponding to an amount exceeding the saturation level D_{MAX} is lost from the signal D_{X}, the signal D_{X}-D_{F} on odd-numbered rows becomes smaller than a value which should be obtained. In contrast, as for the signal D_{X}-D_{F} on even-numbered rows, the signal D_{X} has not reached the saturation level D_{MAX} and no signal component is lost from the signal D_{X}. As a result, the signal D_{X}-D_{F} in Fig. 4B draws the plot in which the rise and fall of the signal value are repeated between odd-numbered rows and even-numbered rows.

That is, when a signal D_{X} near the saturation level D_{MAX} is obtained as in a region of the sensor unit 101 where radiation not having passed through an object X is detected, a portion at which saturated and non-saturated signals are mixed may be generated. This is because noise components contained in signals read out by the readout unit 103 are different from each other between adjacent rows between which the time of the reset operation K1 to readout operation H1 (or the reset operation K2 to readout operation H2) is different. In this case, a striped artifact appears in a radiation image obtained by the radiation imaging apparatus 100. To solve this, a portion which should be corrected is specified and undergoes correction processing by the determination unit 116 and correction unit 117.

The determination unit 116 determines and specifies a portion of a signal read out by the readout unit 103 at which saturated and non-saturated signals are mixed (first processing). Based on the non-saturated signal at this portion, the correction unit 117 corrects the saturated signal at this portion which is specified by the determination of the determination unit 116 and at which the saturated and non-saturated signals are mixed (second processing).

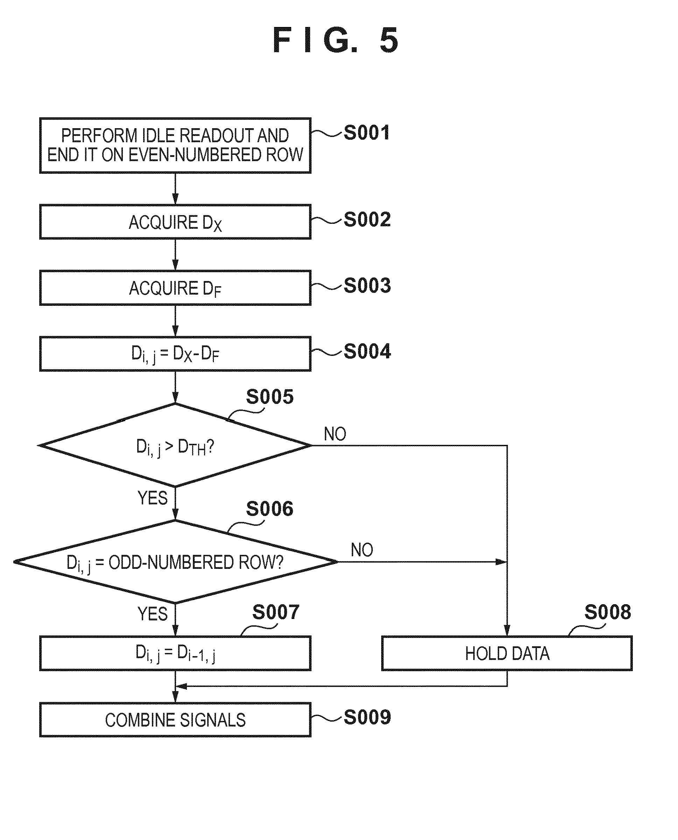

The determination method and correction method will be explained with reference to Fig. 5. First, the determination unit 116 determines on which of odd- and even-numbered rows the reset operations K1 and K2 ended (step S001). A case in which the reset operations K1 and K2 ended on an even-numbered row will be examined. Then, the signal D_{X} is obtained by the readout operation H1 according to the above-mentioned sequence (step S002). A pixel which has output a saturated signal and a pixel which has output a non-saturated signal may be discriminated based on the obtained signal D_{X}.

After that, the signal D_{F} is obtained by the readout operation H2 (step S003). At this time, the absolute value Δdf of the difference between a signal output in the readout operation H2 from a pixel which has output a saturated signal in the readout operation H1, and a signal output in the readout operation H2 from a pixel which has output a non-saturated signal in the readout operation H1 can be calculated. For example, the absolute value Δdf is calculated between adjacent pixels, one of which has output a saturated signal in the readout operation H1 and the other one of which has output a non-saturated signal. Based on the signal D_{F}, an average value D_{AVE} of signals output in the readout operation H2 from pixels which have output non-saturated signals in the readout operation H1 can be calculated.

Then, the processing unit 105 obtains the signal D_{X}-D_{F} (step S004). Of the signals D_{X}-D_{F}, a signal from the ith row and jth column is represented by D_{i,j}. By using a threshold D_{TH} = D_{MAX} - D_{AVE} - Δdf, the determination unit 116 can specify, as a portion at which saturated and non-saturated signals are mixed, a portion of the signal D_{X}-D_{F} that exceeds the threshold D_{TH} (step S005). For D_{i,J} ≤ D_{TH}, this signal can be held in a temporary storage such as a DRAM (step S008). For D_{i,j} > D_{TH}, it is determined which of a signal on an odd-numbered row and a signal on an even-numbered row is D_{i,j} (step S006). In this case, the reset operations K1 and K2 have ended on an even-numbered row. Thus, if D_{i,j} is a signal on an even-numbered row, the process advances to step S008 described above; if D_{i,j} is a signal on an odd-numbered row, to step S007. In step S007, the value of the signal D_{i,j} is changed to the value of a signal on an adjacent even-numbered row (in this case, a signal D_{i-1,j} immediately preceding by one row). Finally, signals on the respective rows are combined (step S009), completing the correction processing.

The arrangement has been exemplified above, in which at a portion which is determined or specified by the determination unit 116 and at which saturated and non-saturated signals are mixed, the correction unit 117 changes the value of the saturated signal into the value of a non-saturated signal obtained from a pixel adjacent to the pixel which has output the saturated signal. However, the correction method is not limited to this. For example, the correction unit 117 may calculate the average value ((D_{i-1,j} + D_{i+1,j})/2) of non-saturated signals output from two pixels adjacent to the pixel which has output the saturated signal, and change the value of the saturated signal into the average value.

The above-described embodiment assumes irradiation with radiation at a uniform irradiation dose for easy understanding. To the contrary, Figs. 6A and 6B exemplify plots when the irradiation dose has a distribution at a predetermined gradient. More specifically, Fig. 6A shows the signals D_{X}, D_{F}, and D_{X}-D_{F} when the intensity of radiation is low. Fig. 6B shows the signals D_{X}, D_{F}, D_{X}-D_{F}, and (D_{X}-D_{F})' when the intensity of radiation is high. As is apparent from Fig. 6B, the signals D_{X} on the seventh row G7 and subsequent odd-numbered rows have reached the saturation level, and plotted values of the signal D_{X}-D_{F} that correspond to the respective rows become smaller than a value which should be obtained. Fig. 6B shows the plot of the signal (D_{X}-D_{F})' corrected by the correction method of changing a signal to the aforementioned average value.

Since the noise component can change depending on the temperature and the time elapsed after the radiation imaging apparatus 100 is activated, the threshold D_{TH} is preferably updated in every imaging (inspection). It is also possible to divide the sensor unit 101 into a plurality of regions, calculate the average value D_{AVE} for each region, and set the threshold D_{TH}.

### (Second Embodiment)

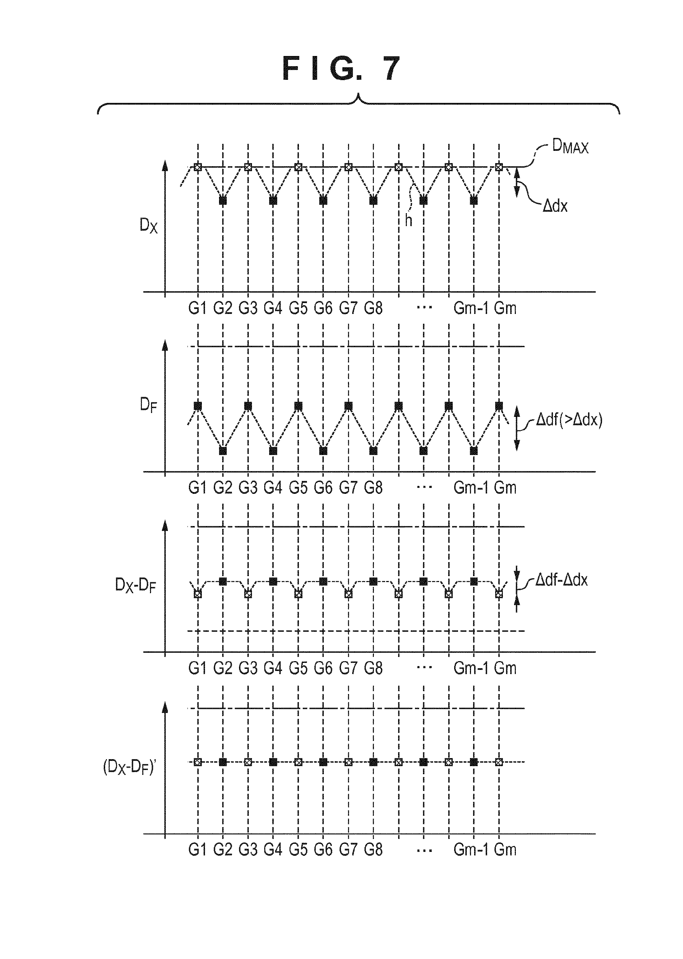

The second embodiment will be described with reference to Fig. 7. The second embodiment will examine a case in which a radiation imaging apparatus 100 is operated according to the same sequence as that in the first embodiment. However, the second embodiment is different from the first embodiment in the method for correcting a signal. Fig. 7 shows a plot similar to that in the first embodiment (Fig. 4B). In the second embodiment, a determination unit 116 determines which of a saturated signal and non-saturated signal is a signal D_{X} obtained by a readout operation H1, and specifies a portion at which these two signals are mixed. At this time, a difference Δdx (absolute value) between saturated and non-saturated signals obtained from two adjacent pixels can be calculated.

Then, a signal obtained by a readout operation H2 can be analyzed. More specifically, a difference Δdf (absolute value) between a signal output in the readout operation H2 from a pixel which has output a saturated signal in the readout operation H1, and a signal output in the readout operation H2 from a pixel which has output a non-saturated signal in the readout operation H1 can be calculated. A correction unit 117 adds Δdf - Δdx to a portion of the obtained signal D_{X}-D_{F} that is obtained from the pixel which has output a saturated signal in the readout operation H1. Even by this correction method, an almost uniformly plotted signal (D_{X}-D_{F})' is obtained.

### (Third Embodiment)

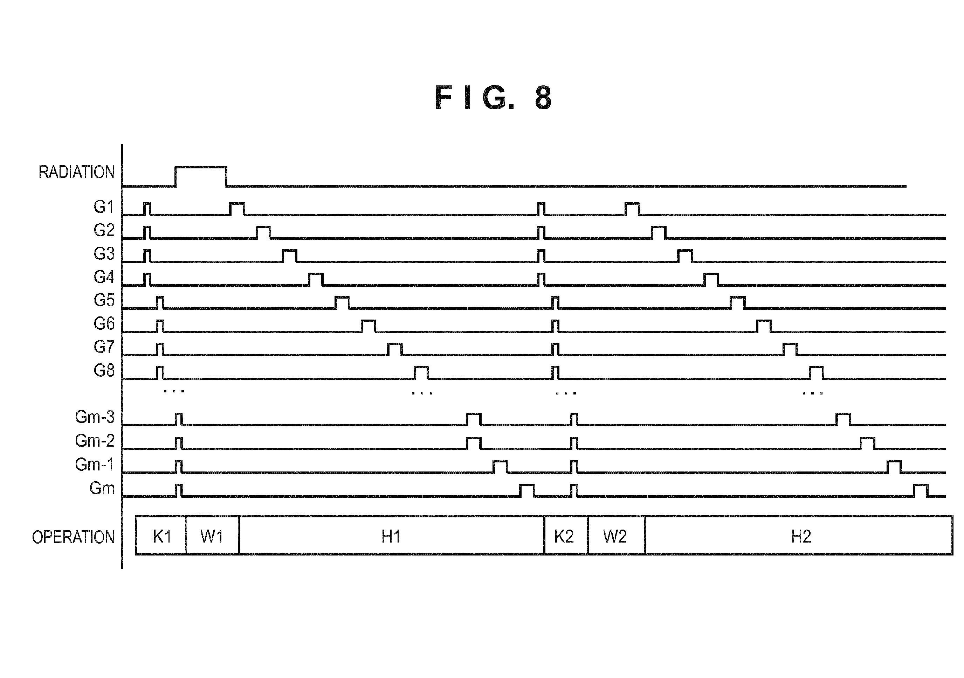

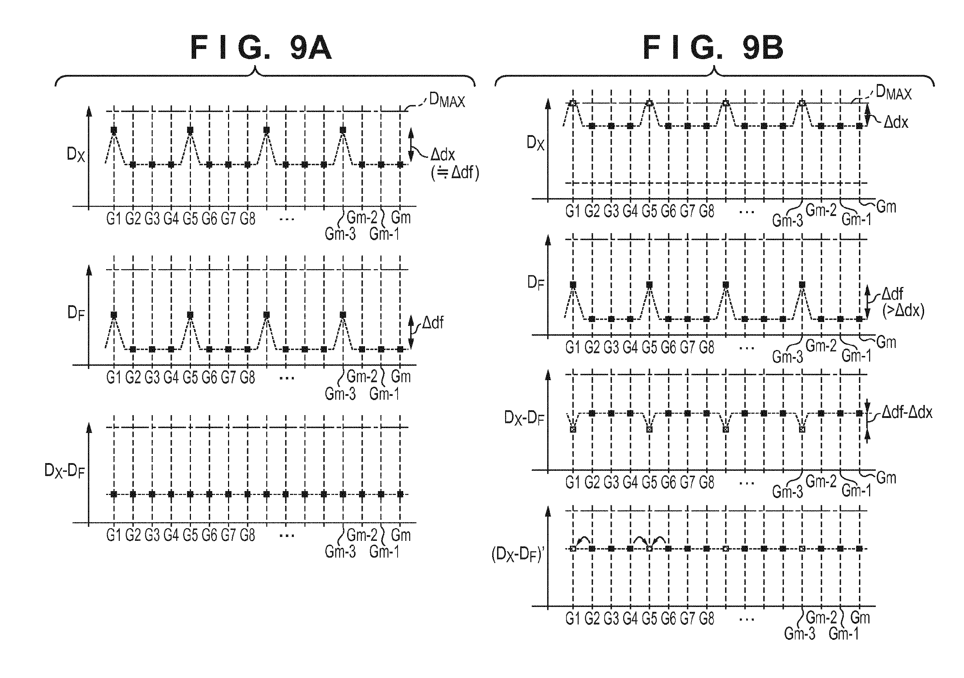

The third embodiment will be described with reference to Figs. 8, 9A, and 9B. The third embodiment is different from the first or second embodiment in that respective pixels P are cyclically reset for every two or more rows, as exemplified in Fig. 8. A case in which the respective pixels P are cyclically reset for every four rows will be exemplified. This reset method improves the responsiveness of radiation detection of a detection unit 120 because the current amount on a bias line Bs increases. Also, this reset method can reduce shading of an image signal because the time difference in standby operation between adjacent rows is decreased.

Even when a radiation imaging apparatus 100 is operated by this reset method, a striped artifact may appear in a radiation image obtained by the radiation imaging apparatus 100. More specifically, when the respective pixels P are cyclically reset for every four rows, a noise component mixed in a signal from a row of a smallest row G number out of these four rows can become larger by, for example, about several hundred LSB, compared to the remaining three rows. Figs. 9A and 9B show the plots of respective signals, similar to Figs. 4A and 4B. More specifically, Fig. 9A shows signals D_{X}, D_{F}, and D_{X}-D_{F} when the intensity of radiation is low. Fig. 9B shows the signals D_{X}, D_{F}, D_{X}-D_{F}, and (D_{X}-D_{F})' when the intensity of radiation is high.

As is apparent from Figs. 9A and 9B, a noise component mixed in a signal from a row of a smallest row G number out of every four rows to be reset becomes larger, compared to the remaining rows. This is because a noise component output from each pixel P in the reset operation contains, for example, the noise component of the substrate of a sensor unit 101, and the noise component is concentrated on a row close to a readout unit 103, out of every four rows to be reset. When, therefore, the intensity of radiation is high, a signal containing a saturated signal is sometimes obtained. This saturated signal is corrected by the same procedures as those in the first or second embodiment.

Although the three embodiments have been described above, the present invention is not limited to them. Changes can be appropriately made in accordance with the purpose, state, application, function, and other specifications, and the present invention can also be implemented by another form. For example, the present invention has been described by exemplifying an arrangement in which the determination unit 116 and correction unit 117 are included in the processing unit 105. However, the present invention is not limited to this arrangement, and the determination unit 116 and correction unit 117 may be included in the external processing unit 109. For example, the above-described signal correction can also be implemented by executing, by a computer, a correction program for performing the above-described correction processing without using the determination unit 116 and correction unit 117. The correction program can be used from a recording medium such as a CD-ROM or a transmission means such as the Internet.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A radiation imaging apparatus (100) comprising:
a pixel array (101) on which a plurality of pixels (S) are arrayed;
a readout unit (103) configured to read out a signal from the pixel array;
a first unit (116) configured to specify a portion of the signal read out by the readout unit, at which a saturated signal whose value has reached a saturation level of an output by the readout unit and a non-saturated signal whose value has not reached the saturation level are mixed; and
a second unit (117) configured to correct, based on the non-saturated signal, the saturated signal at the portion specified by the first unit.

2. An apparatus (100) according to claim 1, further comprising:
a driving unit (102) configured to drive the pixel array; and
a control unit (106),
wherein the control unit controls the driving unit to perform a reset operation of cyclically resetting the respective pixels for every row, and a readout operation of outputting a signal from the pixel array to the readout unit (103), and
in the reset operation, the respective pixels are reset by alternately repeat resetting of pixels arranged on odd-numbered rows and resetting of pixels arranged on even-numbered rows, out of a plurality of rows in the pixel array (101).

3. An apparatus (100) according to claim 1, further comprising:
a driving unit (102) configured to drive the pixel array; and
a control unit (106),
wherein the control unit controls the driving unit to perform a reset operation of cyclically resetting the respective pixels for at least every two rows, and a readout operation of outputting a signal from the pixel array to the readout unit.

4. An apparatus (100) according to claim 2, wherein
the control unit (106) controls the driving unit (102)
to perform a first reset operation,
to interrupt the first reset operation in response to irradiation with radiation,
to perform a first readout operation after accumulating charges in the respective pixels,
to start a second reset operation in response to an end of the first readout operation,
to interrupt the second reset operation on a row on which the first reset operation was interrupted after lapse of a time of at least one cycle of the reset operation, and
to perform a second readout operation after lapse of a time during which the charge accumulation was performed, and
the signal from the pixel array (101) is formed based on a difference between a signal obtained by the first readout operation and a signal obtained by the second readout operation.

5. An apparatus according to claim 4,
wherein in a case where:
D_{MAX} represents the saturation level,
Δdf represents an absolute value of a difference between a signal output in the second readout operation from a pixel which has output the saturated signal in the first readout operation and a signal output in the second readout operation from a pixel which has output the non-saturated signal in the first readout operation, and
D_{AVE} represents an average value of signals output in the second readout operation from pixels each of which has output the non-saturated signal in the first readout operation,
the first unit (116) specify a portion of the signal from the pixel array that exceeds D_{MAX} - D_{AVE}-Δdf.

6. An apparatus according to claim 4,
wherein in a case where:
Δdx represents an absolute value of a difference between the saturated signal and the non-saturated signal obtained in the first readout operation, and
Δdf represents an absolute value of a difference between a signal output in the second readout operation from a pixel which has output the saturated signal in the first readout operation, and a signal output in the second readout operation from a pixel which has output the non-saturated signal in the first readout operation,
the second unit (117) adds Δdf - Δdx to a portion of the signal from the pixel array that is obtained from a pixel which has output the saturated signal in the first readout operation.

7. An apparatus (100) according to claim 1, further comprising:
a bias line configured to supply a predetermined bias to the respective pixels; and
a detection unit configured to monitor a current amount on the bias line and detect irradiation with radiation based on a change amount of the current amount.

8. A radiation inspection apparatus (SYS) comprising:
a radiation imaging apparatus (100) defined in any one of claims 1 to 7;
a processing unit (109) configured to process a signal from the radiation imaging apparatus; and
a radiation source (112) configured to generate radiation.

9. A method for correcting a signal obtained by a radiation imaging apparatus including a pixel array (101) on which a plurality of pixels are arrayed, and a readout unit (103) configured to read out a signal from the pixel array, comprising:
specifying a portion of the signal read out by the readout unit, at which a saturated signal whose value has reached a saturation level of an output by the readout unit and a non-saturated signal whose value has not reached the saturation level are mixed; and
correcting, based on the non-saturated signal, the saturated signal at the specified portion.

10. A program that, when executed by a radiation imaging apparatus (100), causes the radiation imaging apparatus to perform a method according to claim 9.

11. A storage medium storing a program according to claim 10.
